(19) Europäisches Patentamt
European Patent Office
Office européen des brevets

(11) Veröffentlichungsnummer: **0 010 248**
**B1**

# (12) EUROPÄISCHE PATENTSCHRIFT

(45) Veröffentlichungstag der Patentschrift: **15.07.81**

(21) Anmeldenummer: **79103853.2**

(22) Anmeldetag: **08.10.79**

(51) Int. Cl.³: **C 07 C 127/24,** C 07 C 125/06, C 07 D 251/34, C 08 G 18/72, C 08 G 18/80, C 09 D 3/48

## (54) Isocyanatgemisch und seine Verwendung als Bindemittel in Einkomponenten-Lacken.

(30) Priorität: **19.10.78 DE 2845514**

(43) Veröffentlichungstag der Anmeldung: **30.04.80 Patentblatt 80/9**

(45) Bekanntmachung des Hinweises auf die Patenterteilung: **15.07.81 Patentblatt 81/28**

(84) Benannte Vertragsstaaten: **BE DE FR GB IT NL SE**

(56) Entgegenhaltungen:
**BE-A-837 599**
**FR-A-1 455 546**
**FR-A-2 235 145**
**FR-A-2 235 147**

(73) Patentinhaber: **BAYER AG, Zentralbereich Patente, Marken und Lizenzen, D-5090 Leverkusen 1, Bayerwerk (DE)**

(72) Erfinder: **Kubitza, Werner, Eduard-Spranger-Strasse 22, D-5090 Leverkusen 3 (DE)**
Erfinder: **Mennicken, Gerhard, Dr., Am Wasserturm 16, D-5090 Leverkusen 3 (DE)**
Erfinder: **Pedain, Josef, Dr., Haferkamp 6, D-5000 Köln 80 (DE)**

**EP 0 010 248 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Isocyanatgemisch und seine Verwendung als Bindemittel in Einkomponenten-Lacken

Die vorliegende Erfindung betrifft neue, physiologisch unbedenkliche, als Bindemittel in lösungsmittelfreien Einkomponenten-Lacken geeignete Isocyanatgemische und deren Verwendung.

Lösungsmittelarme bzw. -freie Zweikomponenten-Polyurethanlacke sind bekannt (vgl. z.B. DE-B 2006055 oder DE-B 2304893). Nachteilhaft bei diesen Zweikomponenten-Systemen ist insbesondere ihre nur begrenzte Topfzeit.

Einkomponenten-Bindemittel auf Basis organischer Isocyanate, die einerseits in Abwesenheit von Feuchtigkeit praktisch unbegrenzt lagerfähig sind, und die andererseits zur Herstellung von nach allen üblichen Methoden der Lacktechnologie verarbeitbaren, lösungsmittelfreien bzw. -armen, unter dem Einfluss von Luftfeuchtigkeit aushärtenden Lacken verwendet werden können, sind bislang noch nicht bekannt geworden. Die bekannten Einkomponenten-Systeme auf Isocyanat-Basis, d.h. die bekannten NCO-Gruppen aufweisenden Präpolymere weisen im allgemeinen eine zu hohe Viskosität auf, um beispielsweise lösungsmittelfrei durch Spritzen verarbeitbar zu sein.

Es war die Aufgabe der vorliegenden Erfindung, derartige Einkomponenten-Bindemittel zur Verfügung zu stellen. Diese Aufgabe konnte mit den nachstehenden näher beschriebenen Isocyanatgemischen gelöst werden.

Gegenstand der vorliegenden Erfindung ist ein in Abwesenheit von Feuchtigkeit lagerfähiges, unter dem Einfluss von Feuchtigkeit unter Bildung von Harnstoff-Gruppierungen vernetzbares, als Bindemittel für Einkomponenten-Lacke geeignetes Isocyanat-Gemisch einer im DIN-Becher 4 gemäss DIN 53211 gemessenen Viskosität von 30 bis 200 Sekunden und einem Gehalt an physiologisch bedenklichen, leicht flüchtigen organischen Diisocyanaten von maximal 0,7 Gew.-% dadurch gekennzeichnet, dass es ein Gemisch darstellt aus

a) mindestens einem Biuret-, Urethan- und/oder Isocyanurat-Gruppen aufweisenden Lackpolyisocyanat einer über 2 liegenden mittleren NCO-Funktionalität und eines zwischen 13 und 30 Gew.-% liegenden NCO-Gehalts und

b) mindestens eines Monoisocyanats der Formel

$$R_1\text{-O-CO-NH-}R_2\text{-NCO}$$

in welcher

$R_1$ für einen gegebenenfalls durch Ätherbrücken unterbrochenen Kohlenwasserstoffrest mit 1 bis 18 Kohlenstoffatomen steht und

$R_2$ für einen Rest steht, wie er durch Entfernung der Isocyanat-Gruppen aus einem aliphatischen, cycloaliphatischen, araliphatischen oder aromatischen Diisocyanat eines zwischen 140 und 300 liegenden Molekulargewichts erhalten wird,

wobei das Gewichtsverhältnis der Komponenten a):b) gleich oder grösser als 1:1 und die mittlere NCO-Funktionalität des Gemisches grösser als 1,8 sind.

Gegenstand der vorliegenden Erfindung ist auch die Verwendung dieser Isocyanat-Gemische als Bindemittel in, unter dem Einfluss von Feuchtigkeit aushärtbaren, Einkomponenten-Lacken.

Bei der Komponente a) der erfindungsgemässen Gemische handelt es sich um Lackpolyisocyanate, d.h. insbesondere Biuret-, Urethan- oder Isocyanuratgruppen aufweisende Polyisocyanate einer über 2, vorzugsweise zwischen 2,5 und 6 liegenden mittleren NCO-Funktionalität. Diese Polyisocyanate weisen vorzugsweise aromatisch, cycloaliphatisch oder aliphatisch gebundene Isocyanatgruppen auf. Für lichtechte Beschichtungen werden Polyisocyanate mit aliphatisch und/oder cycloaliphatisch gebundenen Isocyanatgruppen bevorzugt. Die erfindungsgemäss einzusetzenden Lackpolyisocyanate werden durch an sich bekannte Modifizierung von einfachen organischen Diisocyanaten unter Biuret-, Urethan- oder Isocyanurat-Bildung hergestellt, wobei jeweils nach der Modifizierungsreaktion eventuell noch vorliegende Überschüsse an nicht modifiziertem monomerem Ausgangsisocyanat in an sich bekannter Weise vorzugsweise durch Destillation so entfernt werden, dass in den erfindungsgemäss einzusetzenden Lackpolyisocyanaten höchstens 0,7, vorzugsweise 0,5 Gew.-% an überschüssigem Diisocyanat vorliegen. Die erfindungsgemäss einzusetzenden Lackpolyisocyanate weisen im allgemeinen einen NCO-Gehalt bezogen auf Feststoff im Bereich von 10 bis 30 Gew.-% und vorzugsweise eine maximale Viskosität von 4000 mPa.s/20 °C auf. Dies entspricht einer Auslaufviskosität von ca. 500 Sekunden im DIN-Becher, 4 mm-Düse, gemäss DIN 53211.

Geeignete Diisocyanate zur Herstellung der Lackpolyisocyanate sind beispielsweise 2,4- und/oder 2,6-Diisocyanatotoluol, 2,4'-Diisocyanatodicyclohexylmethan, 4,4'-Diisocyanato-dicyclohexylmethan, Hexamethylendiisocyanat oder 1-Isocyanato-3,3,5-trimethyl-5-isocyanatomethyl-cyclohexan (IPDI). 2,4-Diisocyanatotoluol, Hexamethylendiisocyanat und IPDI stellen die bevorzugten Diisocyanate zur Herstellung der erfindungsgemäss einzusetzenden Lackpolyisocyanate dar. Die Herstellung der Lackpolyisocyanate aus den beispielhaft genannten Ausgangsdiisocyanaten erfolgt nach bekannten Methoden des Standes der Technik. So kann beispielsweise die Herstellung der Biuretgruppen aufweisenden Lackpolyisocyanate nach dem Verfahren der US-A 3124605, 3358010, 3903126, 3903127 oder 3976622 erfolgen. Die Herstellung von erfindungsgemäss einsetzbaren Urethanpolyisocyanaten geschieht beispielsweise gemäss US-A 3183112, während die Herstellung von Isocyanuratgruppen aufweisenden, erfindungsgemäss geeigneten Lackpolyisocyanaten beispielsweise

nach den Verfahren der GB-A 1 060 430, 1 234 972, 1 506 373 oder 1 458 564 bzw. gemäss US-A 3 394 111, US-A 3 645 979 oder US-A 3 919 218 oder gemäss DE-A 2 839 133 erfolgt.

Zu den besonders bevorzugten Lackpolyisocyanaten gehören die Biuretgruppen aufweisenden Polyisocyanate gemäss US-A 3 124 605, und vor allem US-A 3 903 127, insbesondere jene auf Basis von Hexamethylendiisocyanat, die Urethangruppen aufweisenden Polyisocyanate gemäss US-PS 3 183 112, insbesondere jene auf Basis von 2,4-Diisocyanatotoluol, Trimethylolpropan und den verschiedenen Butandiolen, sowie die Isocyanuratgruppen aufweisenden Polyisocyanate gemäss den obengenannten Patentschriften, insbesondere die entsprechenden Isocyanuratgruppen aufweisenden Polyisocyanate auf Basis von 2,4-Diisocyanatotoluol, dessen Gemischen mit Hexamethylendiisocyanat oder auf Basis von IPDI.

Bei der Komponente b) des erfindungsgemässen Gemisches handelt es sich um Monoisocyanate der Formel

$$R_1-O-CO-NH-R_2-NCO.$$

In dieser Formel haben $R_1$ und $R_2$ die bereits genannte Bedeutung. Vorzugsweise stehen

$R_1$ für einen gegebenenfalls durch Äthersauerstoffatome unterbrochenen, gesättigten aliphatischen Kohlenwasserstoffrest mit 6 bis 12 Kohlenstoffatomen, insbesondere einen gesättigten aliphatischen Kohlenwasserstoffrest mit 6 bis 12 Kohlenstoffatomen und einer verzweigten Kohlenstoffkette und

$R_2$ für einen gesättigten aliphatischen Kohlenwasserstoffrest mit 4 bis 12, insbesondere 6 Kohlenstoffatomen oder einen gesättigten cycloaliphatischen Kohlenwasserstoffrest mit 6 bis 15 Kohlenstoffatomen, wobei jeweils zwischen den beiden Stickstoffatomen der obengenannten Formel mindestens 2 Kohlenstoffatome angeordnet sind.

Diese Monoisocyanate können auf einfache Weise durch Umsetzung von überschüssigen Mengen eines Diisocyanates $R_2$ $(NCO)_2$ mit einer Hydroxylverbindung $R_1$–OH und anschliessende destillative Entfernung des nichtumgesetzten Diisocyanatüberschusses beispielsweise in einem Dünnschichtverdampfer hergestellt werden.

Geeignete Diisocyanate $R_2(NCO)_2$ sind beispielsweise Tetramethylendiisocyanat, Hexamethylendiisocyanat, Dodecanmethylendiisocyanat, 1,3- und 1,4-Diisocyanatocyclohexan, 2,4- und 2,6-Diisocyanatotoluol, 1-Methyl-2,4-diisocyanatocyclohexan, 4,4′-Diisocyanato-dicyclohexylmethan oder 1-Isocyanato-3,3,5-trimethyl-5-isocyanatomethyl-cyclohexan (IPDI).

Bevorzugt findet Hexamethylendiisocyanat Verwendung.

Geeignete Hydroxylverbindungen $R_1$–OH sind beispielsweise: Methanol, Äthanol, i-Propanol-, i-Butanol, n-Dodecanol, n-Octadecanol, Äthoxyäthanol, Äthoxy-äthoxyäthanol, Propoxyäthanol, Cyclohexanol und besonders bevorzugt verzweigte Alkohole wie z.B. Neopentylalkohol, 2-Äthylhexanol oder die isomeren Trimethylhexanole.

Bei der Herstellung der Monoisocyanate werden die Diisocyanate und Alkohole im übrigen so ausgewählt, dass die der obigen Formel entsprechenden Umsetzungsprodukte bei Raumtemperatur flüssige, dünn-viskose Monoisocyanate darstellen.

Sowohl die Komponente a) als auch die Komponente b) der erfindungsgemässen Gemische werden vor der Herstellung der erfindungsgemässen Gemische vorzugsweise von physiologisch bedenklichen, zu ihrer Herstellung eingesetzten flüchtigen Diisocyanaten, beispielsweise mittels eines Dünnschichtverdampfers befreit, so dass der Gehalt an derartigen Diisocyanaten in den erfindungsgemässen Gemischen unter 0,7, vorzugsweise unter 0,5 Gew.-%, liegt.

Die Herstellung der erfindungsgemässen Gemische erfolgt durch einfaches Vermischen der Komponenten a) und b) wobei das Gewichtsverhältnis der Komponente a) zu Komponente b) gleich oder grösser als 1:1, vorzugsweise zwischen 2:1 und 5:1 liegt. Die Mengenverhältnisse der Komponenten a) und b) werden im übrigen so bemessen, dass die Gemische eine über 1,8, vorzugsweise zwischen 2 und 3 liegende mittlere NCO-Funktionalität und eine im DIN-Becher 4 gemäss DIN 53 211 gemessene Viskosität zwischen 30 und 200, vorzugsweise zwischen 60 und 170 Sekunden aufweisen.

Die erfindungsgemässen Gemische stellen dünnviskose Bindemittel dar, die in Abwesenheit von Feuchtigkeit praktisch unbegrenzt lagerfähig und unter dem Einfluss von Feuchtigkeit, insbesondere von Luftfeuchtigkeit, unter Ausbildung von Harnstoffgruppierungen vernetzen. Wegen ihrer niedrigen Viskosität eignen sie sich zur Herstellung von lösungsmittelfreien bzw. -armen Einkomponenten-Lacken. Bei der Herstellung derartiger Lacke kann somit auf die Mitverwendung von Lösungsmitteln verzichtet werden. Gegebenenfalls genügt eine geringe Menge, d.h. 0 bis 15 Gew.-%, bezogen auf Bindemittel, an Lacklösungsmitteln oder Weichmachern, um die gewünschte Ausgangsviskosität des Beschichtungsmittels einzustellen. Geeignete derartige Lacklösungsmittel sind z.B. Äthylacetat, Butylacetat, Methyläthylketon, Methylisobutylketon oder Xylol. Geeignete Weichmacher sind z.B. Dibutylphthalat, Tributylphosphat oder Adipinsäuredimethylester.

Die erfindungsgemässen Gemische können bei der erfindungsgemässen Verwendung mit allen in der Lacktechnologie üblichen Hilfs- und Zusatzmitteln wie z.B. Katalysatoren für die Isocyanat-Wasser-Reaktion, Pigmenten, Füllstoffen, Verlaufmitteln oder Trocknungsmitteln abgemischt werden. Die gebrauchsfertigen Lacke weisen im allgemeinen eine im DIN-Becher 4 gemäss DIN 53 211 gemessene Auslaufviskosität von 20 bis 400, vorzugsweise 50 bis 200 Sekunden, auf. Sie sind wegen der Abwesenheit von leichtflüchtigen Diisocyanaten physiologisch einwandfrei und können nach den üblichen Applikationsmethoden wie z.B. Streichen, Rollen oder Spritzen auf die zu beschichtenden Substrate aufgebracht werden. Sie eignen sich u.a. zur Beschichtung von Metallen, Holz, Kunststoff, Beton, Papier oder Asbestzement. Da die Verarbeitung bevorzugt lösungsmittelfrei erfolgt, treten weder ökologische Probleme noch Geruchsbelästigungen während und nach der Applikation auf. Die Lacke können u.a. für Schutz- und Dekorationsanstriche z.B. in der Lebensmittelindustrie eingesetzt werden, ohne dass eine Gefahr der Geschmacksbeeinflussung gegeben ist.

In den nachfolgenden Beispielen werden folgende Isocyanate verarbeitet:

Monoisocyanat A:

Umsetzungsprodukt von 1 Mol 2-Äthylhexanol mit 3 Mol eines Gemischs aus 80 Teilen 2,4- und 20 Teilen 2,6-Diisocyanatotoluol unter anschliessender Entfernung des Diisocyanat-Überschusses. Das dünnviskose Monoisocyanat weist einen NCO-Gehalt von 14,0 Gew.-% und einen Gehalt an monomerem Diisocyanat von 0,3 Gew.-% auf.

Monoisocyanat B:

Umsetzungsprodukt von 1 Mol 2-Äthylhexanol mit 3 Mol Hexamethylendiisocyanat unter anschliessender destillativer Entfernung des überschüssigen Diisocyanats. Das dünnviskose Monoisocyanat weist einen NCO-Gehalt von 14,1 Gew.-% und einen Gehalt an monomerem Hexamethyldiisocyanat von 0,2 Gew.-% auf.

Polyisocyanat C:

Im wesentlichen aus Tris-(isocyanato-hexyl)-biuret bestehendes Biuretpolyisocyanat-Gemisch, hergestellt durch Biuretisierung von Hexamethylendiisocyanat gemäss US-PS 3 903 127. NCO-Gehalt: 23,5 Gew.-%; Gehalt an freiem Hexamethylendiisocyanat: $<$0,7 Gew.-%; mittlere NCO-Funktionalität $>$3; Viskosität (DIN-Becher 4 gemäss DIN 53 211): 270 Sekunden.

Polyisocyanat D:

336 g Hexamethylendiisocyanat werden mit 1,2 g Tri-n-butylphosphin versetzt und bei 50 bis 60 °C ca. 8 Stunden gerührt. Der NCO-Gehalt ist dann von 49,5% auf 35 bis 36% gesunken. Man stoppt die Reaktion durch Zugabe von 1,5 g Benzoylchlorid und kurzes Anheizen auf 80° und destilliert das dünnflüssige Reaktionsprodukt 2-fach über einen Dünnschichtverdampfer (Vakuum: 0,3 Torr, Umlauftemperatur des Heizmediums: 160 bis 170 °C). Es werden 161 g Destillat und 170 g Oligomerisat mit einem NCO-Gehalt von 21,1% und einem Gehalt an freiem Hexamethylendiisocyanat von weniger als 0,7% erhalten. Die Auslaufviskosität im DIN-Becher 4 gemäss DIN 53 211 liegt bei 160 Sekunden. (1000 mPa.s/20 °C)

Beispiel 1

25 Gew.-Tle Monoisocyanat A, 25 Gew.-Tle Monoisocyanat B und 100 Gew.-Tle Polyisocyanat C werden vermischt. Das Gemisch weist eine im DIN-Becher 4 gemäss DIN 53 211 gemessene Viskosität von 165 Sekunden, eine über 1,8 liegende mittlere NCO-Funktionalität und einen NCO-Gehalt von 20,3 Gew.-% auf. Nach Zusatz von 0,75 Gew.-Tln Dibutylzinndilaurat als Katalysator erhält man einen in Abwesenheit von Feuchtigkeit unbegrenzt lagerfähigen Klarlack, der durch Rollen zu einem über Nacht mit Luftfeuchtigkeit trocknenden Lackfilm verarbeitet werden kann. Der Lackfilm weist eine Härte gemäss DIN 53 157 von ca. 65 Sekunden und eine Erichsentiefung gemäss DIN 53 156 von 9 mm auf.

Nach Zusatz von 15 Gew.-Tln Adipinsäuredimethylester zu dem obengenannten Gemisch entsteht ein Klarlack einer Viskosität gemäss DIN 53 211 von 70 Sekunden, der nach dem «airless»-Spritzverfahren verarbeitbar ist.

Beispiel 2

100 Gew.-Tle Polyisocyanat C und 50 Gew.-Tle Monoisocyanat B werden vermischt. Das Gemisch weist eine im DIN-Becher 4 gemäss DIN 53 211 gemessene Viskosität von 110 Sekunden, eine mittlere NCO-Funktionalität von $>$1,8 und einem NCO-Gehalt von 20,4 Gew.-% auf. Nach Zusatz von 0,75 Gew.-Tln Dibutylzinndilaurat als Katalysator entsteht ein Klarlack, der in Abwesenheit von Feuchtigkeit unbegrenzt verarbeitbar bleibt und über Nacht bei Raumtemperatur zu einem zäh-elastischen Film aushärtet. Der ausgehärtete Film weist eine Pendelhärte gemäss DIN 53 157 von ca. 60 Sekunden und eine Erichsentiefung gemäss DIN 53 156 von 10 mm auf.

Ohne Zusatz des Monoisocyanats B läge ein Klarlack vor, der wegen seiner hohen Viskosität weder durch Streichen noch durch Rollen applizierbar ist.

Beispiel 3

100 Gew.-Tle Polyisocyanat C werden mit 50 Gew.-Tln Monoisocyanat B vermischt. Das Gemisch weist eine im DIN-Becher 4 gemäss DIN 53 211 gemessene Viskosität von 110 Sekunden, einen NCO-Gehalt von 20,4 Gew.-% und eine mittlere NCO-Funktionalität von $>$1,8 auf. Nach Zusatz von 45 Gew.-Tln Titandioxid (Rutil) 5,4 Gew.-Tle Tosylisocyanat (Trocknungsmittel für das Pigment) und 0,75 Gew.-Tln Dibutylzinndilaurat entsteht eine streich- und rollfähige, lösungsmittelfreie, feuchtigkeitshärtende Einkomponenten-Beschichtungsmasse mit einer Viskosität von 170 Sekunden gemäss DIN 53 211. Der Lack ist unbegrenzt verarbeitbar und trocknet bei Raumtemperatur zu einem zäh-elastischen Film. Der Film weist eine Pendelhärte nach DIN 53 157 von 55 Sekunden und eine Erichsentiefung gemäss DIN 53 156 von 10 mm auf.

Durch Zusatz von 10 Gew.-Tln Adipinsäuredimethylester entsteht eine Beschichtungsmasse einer Viskosität gemäss DIN 53 211 von ca. 95 Sekunden.

Ohne Zusatz des Monoisocyanats B würde das pigmentierte Gemisch selbst bei einer Reduktion des Pigment-Anteils auf 30 Gew.-Tle noch eine

über 5 Minuten liegende Viskosität (DIN 53 211) aufweisen und weder durch Streichen noch durch Rollen applizierbar sein.

Beispiel 4

Jeweils 100 Gew.-Tle Monoisocyanat B, Polyisocyanat C und Polyisocyanat D werden vermischt. Das Gemisch weist eine Viskosität gemäss DIN 53 211 von 60 Sekunden, eine mittlere NCO-Funktionalität von >1,8 und einen NCO-Gehalt von 19,7 Gew.-% auf. Nach Zusatz von 150 Gew.-Tln Titandioxid (Rutil), 18 Gew.-Tln Tosylisocyanat und 1,5 Gew.-Tln Dibutylzinndilaurat entsteht eine streich- und rollfähige, lösungsmittelfreie feuchtigkeitshärtende Einkomponenten-Beschichtungsmasse mit einer Auslaufviskosität von ca. 110 Sekunden nach DIN 53 211. Nach Zusatz von 30 Gew.-Tln Adipinsäuredimethylester entsteht eine Beschichtungsmasse einer gemäss DIN 53 211 gemessenen Viskosität von ca. 55 Sekunden. Diese Beschichtungsmasse ist dann u.a. durch «airless»-Spritzen verarbeitbar.

Ohne den Zusatz des Monoisocyanats und des Weichmachers entstünde selbst bei einer Reduktion des Pigment-Anteils auf 100 Gew.-Tle ein Gemisch mit einer Auslaufviskosität von >300 Sekunden, das weder durch Streichen noch durch Rollen applizierbar sein würde.

**Patentansprüche**

1. In Abwesenheit von Feuchtigkeit lagerfähiges, unter dem Einfluss von Feuchtigkeit unter Bildung von Harnstoff-Gruppierungen vernetzbares, als Bindemittel für Einkomponenten-Lacke geeignetes Isocyanat-Gemisch einer im DIN-Becher 4 gemäss DIN 53 211 gemessenen Viskosität von 30 bis 200 Sekunden und einem Gehalt an physiologisch bedenklichen leicht flüchtigen organischen Diisocyanaten von maximal 0,7 Gew.-%, dadurch gekennzeichnet, dass es ein Gemisch darstellt aus

a) mindestens einem Biuret-, Urethan- und/oder Isocyanurat-Gruppen aufweisenden Lackpolyisocyanat einer über 2 liegenden mittleren NCO-Funktionalität und eines zwischen 13 und 30 Gew.-% liegenden NCO-Gehalts und

b) mindestens eines Monoisocyanats der Formel

$R_1$-O-CO-NH-$R_2$-NCO

in welcher

$R_1$ für einen gegebenenfalls durch Ätherbrücken unterbrochenen Kohlenwasserstoffrest mit 1 bis 18 Kohlenstoffatomen steht und

$R_2$ für einen Rest steht, wie er durch Entfernung der Isocyanat-Gruppen aus einem aliphatischen, cycloaliphatischen, araliphatischen oder aromatischen Diisocyanat eines zwischen 140 und 300 liegenden Molekulargewichts erhalten wird

wobei das Gewichtsverhältnis der Komponenten a):b) gleich oder grösser als 1:1 und die mittlere NCO-Funktionalität des Gemischs grösser als 1,8 sind.

2. Verwendung von Isocyanat-Gemischen gemäss Anspruch 1, als Bindemittel in, unter dem Einfluss von Feuchtigkeit aushärtbaren, Einkomponenten-Lacken.

**Claims**

1. An isocyanate mixture suitable for use as a binder for one-component lacquers, which mixture is stable in storage in the absence of moisture and is cross-linked by moisture with the formation of urea groups and has a viscosity measured in a DIN cup 4 according to DIN 53 211 of from 30 to 200 seconds and contains a maximum of 0.7% by weight of physiologically harmful, readily volatile organic diisocyanates, characterised in that it consists of a mixture of:

a) at least one lacquer polyisocyanate containing biuret, urethane and/or isocyanurate groups and having an average isocyanate functionality greater than 2 and an isocyanate content of from 13 to 30% by weight and

b) at least one monoisocyanate of the formula

$R_1$-O-CO-NH-$R_2$-NCO

wherein

$R_1$ represents a hydrocarbon group having from 1 to 18 carbon atoms optionally interrupted by ether bridges and

$R_2$ represents a group such as is obtained by removal of the isocyanate groups from an aliphatic, cycloaliphatic, araliphatic or aromatic diisocyanate having a molecular weight of from 140 to 300,

the proportion by weight of component a) to component b) being equal to, or greater than, 1:1, and the average isocyanate functionality of the mixture being greater than 1.8.

2. Use of the isocyanate mixtures according to Claim 1 as binders in one-component lacquers which can be hardened under the influence of moisture.

**Revendications**

1. Mélange d'isocyanates apte à la conservation en absence d'humidité, réticulable sous l'influence de l'humidité avec formation de groupements urée, approprié comme agent liant pour les vernis à un seul composant, ayant une viscosité (mesurée dans la coupe n° 4 suivant la norme DIN 53 211) de 30 à 200 secondes et ayant une teneur maximale de 0,7% en poids en diisocyanates organiques aisément volatils et physiologiquement délicats, caractérisé en ce qu'il comprend:

a) au moins un polyisocyanate de vernis comportant des groupes biuret, uréthane et/ou isocyanurate, ce polyisocyanate ayant une fonctionnalité NCO moyenne supérieure à 2 et une teneur en groupes NCO se situant entre 13 et 30% en poids, et

b) au moins un monoisocyanate de formule:

$R_1$-O-CO-NH-$R_2$-NCO

dans laquelle

$R_1$ représente un radical d'hydrocarbure contenant 1 à 18 atomes de carbone et éventuellement interrompu par des ponts éthers, et

$R_2$ représente un radical obtenu par élimination des groupes isocyanate d'un diisocyanate aliphatique, cycloaliphatique, araliphatique ou aromatique d'un poids moléculaire se situant entre 140 et 300,

le rapport pondéral entre les composants (a) et (b) étant égal ou supérieur à 1:1, tandis que la fonctionnalité NCO moyenne du mélange est supérieure à 1,8.

2. Utilisation de mélanges d'isocyanates suivant la revendication 1 comme agents liants dans des vernis à un seul composant durcissables sous l'influence de l'humdité.